# EUROPEAN PATENT SPECIFICATION

(11) **EP 1 187 833 B1**
(45) Date of publication and mention of the grant of the patent: **06.06.2007**
(21) Application number: 00942115.7
(22) Date of filing: 21.06.2000
(51) Int. Cl.: C07D 417/06, C07D 401/06, C07C 257/22, C07C 277/08, C07C 261/00, C07D 213/61, C07D 277/36, C07D 307/14

(54) **METHOD OF PRODUCING THIAMETHOXAM**
VERFAHREN ZUR HERSTELLUNG VON THIAMETHOXAM
PROCEDE DE FABRICATION DE THIAMETHOXAM

(30) Priority: 23.06.1999 CH 117199
(43) Date of publication of application: 20.03.2002
(73) Proprietor: Syngenta Participations AG, 4058 Basel (CH)
(72) Inventor: SEIFERT, Gottfried, CH-4312 Magden (CH); RAPOLD, Thomas, CH-4323 Wallbach (CH); GISIN, Verena, CH-5028 Ueken (CH)
(74) Representative: Waterman, John Richard
(86) International application number: PCT/EP2000/005762
(87) International publication number: WO 2001/000623

(56) References cited:
- DATABASE CHEMABS [Online] CHEMICAL ABSTRACTS SERVICE, COLUMBUS, OHIO, US; ZHOU, JINGYAO ET AL: "Substitution reaction of dicyandiamide in the presence of phase transfer catalyst" retrieved from STN Database accession no. 126:74527 XP002152387 & CHIN. SCI. BULL. (1996), 41(15), 1263-1265 ,
- PATENT ABSTRACTS OF JAPAN vol. 1995, no. 11, 26 December 1995 (1995-12-26) -& JP 07 224062 A (NIPPON BAYERAGROCHEM KK), 22 August 1995 (1995-08-22)
- GOBEL, THOMAS ET AL: "Synthetic approaches towards CGA 293'343: a novel broad-spectrum insecticide" PESTIC. SCI., vol. 55, no. 3, 1999, pages 355-357, XP000960525

## Description

The present invention relates to a novel type of method of producing 3-(2-chloro-1,3-thiazol-5-ylmethyl)-5-methyl-1,3,5-oxadiazinan-4-yledene(nitro)amine (thiamethoxam). It is known that in order to produce substituted nitroguanidines, nitroenamines or cyano-enamines, a further substituent may be introduced (e.g. by alkylation) into those compounds that may already be substituted once to several times (see e.g. EP patent application 0.375.907). Owing to the presence of several hydrogen atoms in the educts used as the starting material in these reactions, the previously proposed substitution reactions of this kind are often non-selective and lead to undesired substitution products. The aforementioned EP patent applications describe by way of example the production of 1,3-disubstituted 2-nitroguandines by reacting monosubstituted nitroisothioureas with primary amines whilst cleaving mercaptan. However, these nitroisothiourea compounds, containing alkylthio leaving groups, which are proposed as starting compounds in the known processes, can only be obtained with difficulty. In EP-A-0-483,062, a process for the production of related compounds by hydrolysis of hexahydrotriazines is also described. JP 07 224062-A describes a process for preparing thiamethoxam from 5-methyl-4-nitroiminotetrahydro-1,3,5-oxadiazine and 2-chloro-5-chloromethylthiazole using a phase transfer catalyst. The reaction is carried out in methylene chloride with the addition of aqueous sodium hydroxide solution.

It has now been shown that the above-described methods of producing thiamethoxam do not satisfy the requirements demanded of a chemical production process, such as availability, toxicity, stability in storage and purity of the starting materials and excipients, reaction time, energy consumption and volumes yielded by the process, quantity and recovery of the accruing by-products and waste products, as well as purity and yield of the end product. There is therefore a need to provide an improved method of producing this compound.
Accordingly, it is the aim of the present invention to provide an improved method of producing thiamethoxam from readily obtainable starting compounds, which allows specific substitution without obtaining major amounts of undesired by-products. Accordingly, the invention relates to a process for the preparation of the compound of the formula 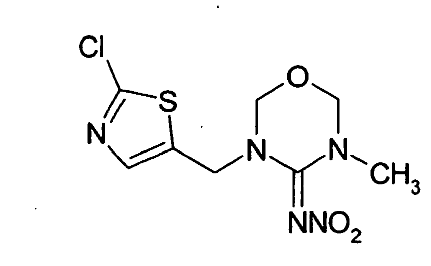 (thiamethoxam, known from EP-A-580533)
which process comprises reacting the compound of formula 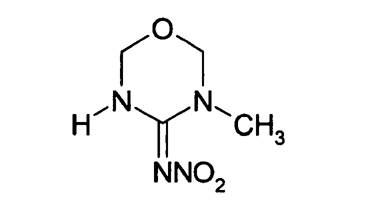 with a compound of the formula 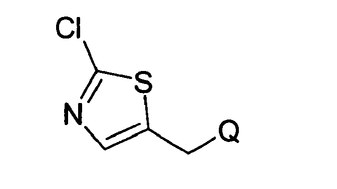 wherein Q is chlorine , in the presence of a solvent or diluent, a phase transfer catalyst and a base, the solvent or diluent being an ester of carbonic acid, the phase transfer catalyst being a quaternary ammonium salt and the base being a carbonate.

Thiamethoxam may be present as salts. Having at least one basic centre it may form e.g. acid addition salts. These are formed for example with strong inorganic acids, such as mineral acids, e.g. sulphuric acid, a phosphoric acid or a hydrohalic acid, with strong organic carboxylic acids, such as C₁-C₄ alkanecarboxylic acids substituted where appropriate for example by halogen, e.g. acetic acid, such as optionally unsaturated dicarboxylic acids, e.g. oxalic, malonic, maleic, fumaric or phthalic acid, such as hydrocarboxylic acids, e.g. ascorbic, lactic, malic, tartaric or citric acid, or benzoic acid, or with organic sulphonic acids, such as C₁-C₄ alkanesulphonic or anylsulphonic acids substituted where appropriate for example by halogen, e.g. methanesulphonic or p-toluenesulphonic acid. Salts of thiamethoxam with acids of the said kind are preferably obtained when working up the reaction mixtures.

In a broader sense, thiamethoxam with at least one acid group can form salts with bases. Suitable salts with bases are for example metal salts, such as alkali or alkaline earth metal salts, e.g. sodium, potassium or magnesium salts, or salts with ammonia or an organic amine, such as morpholine, piperidine, pyrrolidine, a mono-, di- or tri-lower alkylamine, e.g. ethyl-, diethyl-, triethyl- or dimethylpropylamine, or a mono-, di- or trihydroxy-loweralkylamine, e.g. mono-, di- or triethanolamine. Corresponding internal salts where appropriate may also be formed. Preferred compounds within the scope of this invention are agrochemically advantageous salts. Hereinbefore and hereinafter, the free compound, thiamethoxam, is understood where appropriate to include also by analogy the corresponding salts, and the salts are understood to include also the free compound thiamethoxam. The same applies to E/Z isomers and tautomers of thiamethoxam and salts thereof. The free form is preferred.

The phase transfer catalyst is a quaternary ammonium salt as listed in the paper "Phase Transfer Catalysts" by the company Fluka, Buchs, Switzerland, 1986 edition, pages 7 to 25. The quaternary ammonium salts named therein are thus included by reference in the present invention.

Especially preferred quaternary ammonium salts are for example benzyltrimethyl ammonium chloride, benzyltriethyl ammonium chloride, benzyltributyl ammonium chloride, benzyltriethyl ammonium bromide, benzyltrimethyl ammonium methoxide, benzyltrimethyl ammonium hydroxide (triton B), glycidyl trimethyl ammonium chloride, hexadecyl-trimethyl ammonium chloride, hexadecyl-trimethyl ammonium bromide, hexadecyl-pyridinium bromide, hexadecyl-pyridinium chloride, 2-hydroxyethyl-trimethylammonium chloride, 2-hydroxyethyl-trimethylammonium hydroxide, phenyltrimethylammonium chloride, phenyltrimethyl ammonium hydroxide, tetrabutyl ammonium chloride, tetrabutyl ammonium bromide, tetrabutyl ammonium hydroxide, tetrabutyl ammonium tetrafluoroborate, tetrabutyl ammonium nitrate, tetradecyl ammonium chloride, tetradodecyl-ammonium acetate, tetraethyl ammonium chloride, tetraethyl ammonium hydroxide, tetradodecylammonium nitrate, tetradodecyl ammonium toluene sulphonate, tetrahexyl ammonium chloride, tetrahexylammonium bromide, tetramethyl ammonium chloride, tetramethyl-ammonium bromide, tetramethyl ammonium hydroxide, tetramethyl ammonium iodide, tetramethyl ammonium toluene sulphonate, tetraoctyl ammonium chloride, tetrapropyl ammonium chloride, tetrapropyl ammonium bromide, tributylmethyl ammonium chloride and tributylheptyl ammonium bromide, most preferably quaternary ammonium hydroxides, particularly tetramethyl ammonium hydroxide in the form of the pentahydrate.

The solvent or diluent for carrying out the process according to the invention is an ester of carbonic acid.

Particularly preferred solvents are dimethyl carbonate and diethyl carbonate, in particular dimethyl carbonate.

An especially preferred combination is dimethyl carbonate as the solvent with tetramethyl-ammonium hydroxide as the phase transfer catalyst.

The base is a carbonate. Potassium carbonate is preferred. The amount of base employed is preferably one to two moles per mole of the compound of the formula:

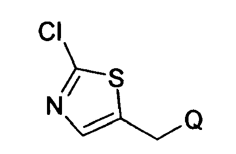

The reaction is dependent on the boiling point of the solvent employed. An advantageous temperature range lies between ca. 40°C and ca. 100°C, preferably between ca. 60°C and ca. 70°C.

A reaction time of ca. 0.1 to ca. 24 hours is preferred, especially ca. 3 to ca. 5 hours.

It has now surprisingly been found that the process according to the invention is able to satisfy to a large extent the requirements listed initially, especially those relating to purity of the produced material.

In particular, it has been shown that, when carrying out the process according to the invention, the formation of undesired isomers can be suppressed. It has been shown that the substitution can also take place on the nitrogen which bears the nitro group:

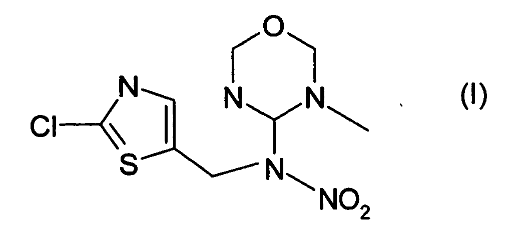

The employment of a phase transfer catalyst permits the use of a solvent, in which only small amounts of undesired isomers are obtained and which may be readily regenerated.

### Preparation Examples

### P1: Preparation of 5-(2-chlorothiazol-5-ylmethyl)-3-methyl-4-nitroimino-perhydro-1,3,5-oxadiazine (Thiamethoxam)

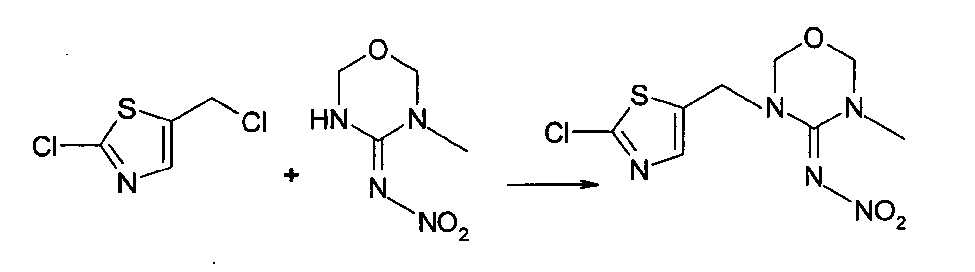

184 g of 100% 3-methyl-4-nitroimino-perhydro-1,3,5-oxadiazine in 400 g of dimethyl carbonate are placed in a sulphonation flask, and 168 g of 100% 2-chloro-5-chloromethylthiazole (1.0 moles) are added as a melt. This mixture is heated to 65°C. A mixture consisting of 350 g of dimethyl carbonate, 4 g of tetramethylammonium hydroxide pentahydrate and 242 g of potassium carbonate powder is measured in whilst stirring over 60 minutes at 62 to 68°C.

The reaction mixture is held for 5 to 6 hours whilst stirring vigorously, until more than 99% of the 2-chloro-5-chloromethylthiazole has reacted (LC control).

The reaction mixture is subsequently cooled to 45-50°C and mixed with 600 g of water. The reaction mixture is adjusted to pH 6.5 with ca. 260 g of 32% hydrochloric acid and is then heated to 60 to 65°C until everything dissolves. The solution is left to stand until phase separation takes place, and the organic phase is separated. The aqueous phase is re-extracted at 50°C with 300 g dimethyl carbonate.

The organic phase from re-extraction is combined with the organic phase from the reaction mixture. The combined organic phases are concentrated under vacuum (350-400 mbar) at 60 to 65°C to a final weight of 600 g (480 ml). The mixture is slowly cooled to 0-5°c and held for 1 hour. Then the resulting suspension is filtered.

The filter cake is washed with 300g of dimethyl carbonate of 5-10°C in two portions and then with 300 ml of water in two portions, and the moist product is dried in a vacuum at 70°C.

Yield 218-220 g of title product in a purity of 98 to 99% (74% of theory based on 100% 2-chloro-5-chloromethylthiazole). The above-mentioned isomer of formula (I) is not found.

The title product may be obtained in a purity of 99.5% by recrystallisation from dimethyl carbonate.

An alternative preparation method comprises adding together the potassium carbonate, 3-methyl-4-nitroimino-perhydro-1,3,5-oxadiazine and the tetramethylammonium hydroxide pentahydrate in 1100 g of dimethyl carbonate, and measuring in the 2-chloro-5-chloromethylthiazole over 60 minutes at 65°C. The subsequent reaction and working up are then carried out as above.

## Claims

1. A process for the preparation of the compound of the formula 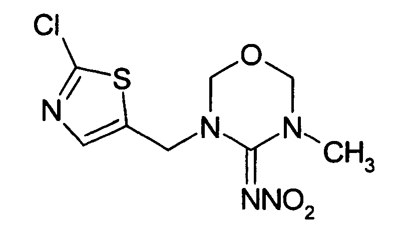 which process comprises reacting the compound of formula 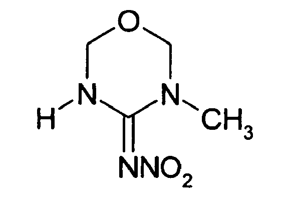 with a compound of the formula 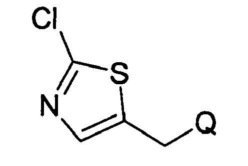 wherein Q is chlorine, in the presence of a solvent or diluent, a phase transfer catalyst and a base, the solvent or diluent being an ester of carbonic acid, the phase transfer catalyst being a quaternary ammonium salt and the base being a carbonate.

2. A process according to claim 1 wherein the phase transfer catalyst is a quaternary ammonium hydroxide.

3. A process according to claim 2 wherein the phase transfer catalyst is tetramethyl ammonium hydroxide in the form of the pentahydrate.

4. A process according to any one of the preceding claims wherein the solvent or diluent is dimethyl carbonate or diethyl carbonate.

5. A process according to claim 4 wherein the solvent or diluent employed is dimethyl carbonate.

6. A process according to claim 1 wherein the solvent or diluent is dimethyl carbonate and the phase transfer catalyst is tetramethyl ammonium hydroxide.

7. A process according to claim any one of the preceding claims wherein the base is potassium carbonate.

## Patentansprüche

1. Verfahren zur Herstellung der Verbindung der Formel 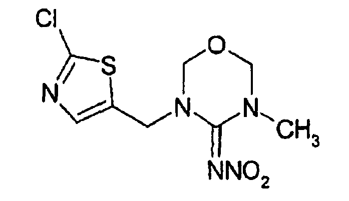 wobei das Verfahren Umsetzen der Verbindung der Formel 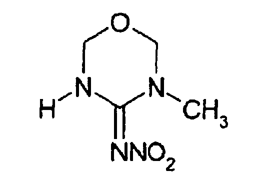 mit einer Verbindung der Formel 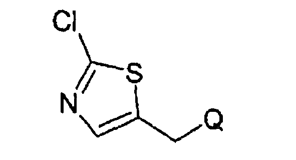 worin Q Chlor darstellt, in Gegenwart eines Lösungsmittels oder Verdünnungsmittels, eines Phasentransferkatalysators und einer Base, wobei das Lösungsmittel oder Verdünnungsmittel einen Ester von Kohlensäure darstellt, der Phasentransferkatalysator ein quaternäres Ammoniumsalz darstellt und die Base ein Carbonat darstellt, umfasst.

2. Verfahren nach Anspruch 1, wobei der Phasentransferkatalysator ein quaternäres Ammoniumhydroxid ist.

3. Verfahren nach Anspruch 2, wobei der Phasentransferkatalysator Tetramethylammoniumhydroxid in Form des Pentahydrats ist.

4. Verfahren nach einem der vorangehenden Ansprüche, wobei das Lösungsmittel oder Verdünnungsmittel Dimethylcarbonat oder Diethylcarbonat ist.

5. Verfahren nach Anspruch 4, wobei das angewendete Lösungsmittel oder Verdünnungsmittel Dimethylcarbonat ist.

6. Verfahren nach Anspruch 1, wobei das Lösungsmittel oder Verdünnungsmittel Dimethylcarbonat ist und der Phasentransferkatalysator Tetramethylammoniumhydroxid ist.

7. Verfahren nach einem der vorangehenden Ansprüche, wobei die Base Kaliumcarbonat ist.

## Revendications

1. Procédé pour la préparation du composé de formule 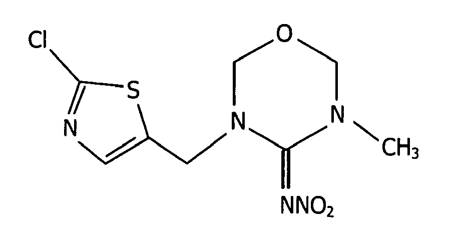 lequel procédé comprend de faire réagir le composé de formule 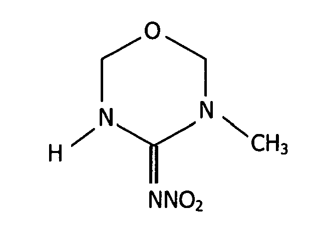 avec un composé de formule 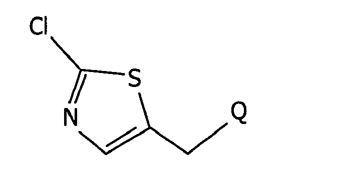 dans laquelle Q est un chlore, en présence d'un solvant ou diluant, d'un catalyseur de transfert de phase et d'une base, le solvant ou diluant étant un ester de l'acide carbonique, le catalyseur de transfert de phase étant un sel d'ammonium quaternaire et la base étant un carbonate.

2. Procédé selon la revendication 1 dans lequel le catalyseur de transfert de phase est un hydroxyde d'ammonium quaternaire.

3. Procédé selon la revendication 2 dans lequel le catalyseur de transfert de phase est l'hydroxyde de tétraméthylammonium sous la forme pentahydratée.

4. Procédé selon l'une quelconque des revendications précédentes dans lequel le solvant ou diluant est le carbonate de diméthyle ou le carbonate de diéthyle.

5. Procédé selon la revendication 4 dans lequel le solvant ou diluant employé est le carbonate de diméthyle.

6. Procédé selon la revendication 1 dans lequel le solvant ou diluant est le carbonate de diméthyle et le catalyseur de transfert de phase est l'hydroxyde de tétraméthylammonium.

7. Procédé selon l'une quelconque des revendications précédentes dans lequel la base est le carbonate de potassium.
